# FASCICULE DE BREVET EUROPEEN

(11) **EP 3 681 527 B1**
(45) Date de publication et mention de la délivrance du brevet: **10.11.2021**
(21) Numéro de dépôt: 18769649.7
(22) Date de dépôt: 11.09.2018
(51) Int. Cl.: A61K 38/17, A61K 8/64, A61P 39/00, A61Q 17/00, A61Q 17/04, A61P 17/00, A61P 17/16

(54) **UTILISATION DE LA SCLÉRITINE EN TANT QU'AGENT PROTECTEUR DE CELLULES CONTRE DES AGENTS TOXIQUES**
VERWENDUNG VON SCLERITIN ALS ZELLSCHUTZAGNZ GEGEN TOXISCHEN MITTELN
USE OF SCLERITIN AS AN AGENT FOR PROTECTING CELLS AGAINST TOXIC AGENTS

(30) Priorité: 11.09.2017 FR 1758347
(43) Date de publication de la demande: 22.07.2020
(73) Titulaire: Centre Scientifique de Monaco, 98000 Monaco (MC); Coraliotech, 98000 Monaco (MC)
(72) Inventeur: BENCHAOUIR, Rachid, 92120 Montrouge (FR); BUCLEZ, Pierre-Olivier, 78950 Elancourt (FR)
(74) Mandataire: Hautier IP
(86) Numéro de dépôt international: PCT/EP2018/074455
(87) Numéro de publication internationale: WO 2019/048703

(56) Documents cités:
- CN-A- 1 346 638
- DEBREUIL JULIEN ET AL: "Molecular Cloning and Characterization of First Organic Matrix Protoein from Sclerites of Red Coral, Corallium rubrum", JOURNAL OF BIOLOGICAL CHEMISTRY, vol. 287, no. 23, juin 2012 (2012-06), pages 19367-19376, XP002782089, cité dans la demande
- RAHMAN M AZIZUR: "An Overview of the Medical Applications of Marine Skeletal Matrix Proteins", MARINE DRUGS, vol. 14, no. 9, 167, septembre 2016 (2016-09), pages 1-9, XP002782090, ISSN: 1660-3397

## Description

### DOMAINE TECHNIQUE

La présente invention concerne la Scléritine et son utilisation en tant qu'agent protecteur contre des agents toxiques.

Le domaine de l'invention concerne l'industrie pharmaceutique ou l'industrie cosmétique et plus spécifiquement pour la formulation de compositions pharmaceutiques ou cosmétiques destinées à protéger les cellules contre des agents toxiques.

### ETAT DE LA TECHNIQUE

Le domaine de la cosmétique est en constante recherche de nouvelles molécules ayant des effets cosmétiques ou dermatologiques. La demande du marché est de plus en plus orientée vers des produits naturels respectueux de l'environnement et de l'homme.

Les coraux sont des organismes dont la physiologie cellulaire et moléculaire est encore assez peu connue et qui vivent individuellement jusqu'à plusieurs décennies. Ils survivent sur de longues périodes, bien que constamment exposés aux attaques bactériennes, virales, fongiques, mais également aux aléas environnementaux de types climatiques ou liés à la pollution ; de nombreux organismes parasites ou prédateurs opportunistes ou spécialisés constituent également une nuisance. La résistance des coraux à l'encontre de l'ensemble de ces effets nocifs préfigure donc une source potentielle de nouvelles molécules naturelles ayant des effets intéressants dans le domaine de la cosmétique, de la dermatologie, ou de la pharmaceutique.

La Scléritine est une protéine qui a été isolée et caractérisée pour la première fois en 2012 comme décrite dans la publication Debreuil et al. "Molecular cloning and characterization of first organic matrix protein from sclerites of red coral, Corallium rubrum J Biol Chem. 2012 Jun 1; 287 (23): 19367-76. Epub 2012 Apr 13.

Cette protéine est extraite de la matrice organique des sclérites de l'octocoralliaire, *Corallium rubrum*, plus communément dénommé corail rouge. La Scléritine est la protéine majoritaire de la fraction soluble dans l'EDTA de la matrice organique. La Scléritine est une protéine basique phosphorylée sécrétée possédant une séquence de 135 acides aminés et un peptide signal de 20 acides aminés.

### EXPOSE DE L'INVENTION

La présente invention propose l'utilisation de la Scléritine comme agent protecteur de cellules contre des agents toxiques, c'est à dire l'invention concerne la Scléritine pour son utilisation dans un traitement thérapeutique, avantageusement dermatologique, en tant qu'agent protecteur pour des cellules contre des agents toxiques.

L'invention concerne la Scléritine pour son utilisation pour la prévention et la protection des cellules contre des agents toxiques.

De manière surprenante, cette protéine issue d'un corail présente une activité protectrice des cellules contre des agents toxiques.

De manière avantageuse, les agents toxiques sont de différents types tels que des agents chimiques ou des agents physiques.

Suivant un autre aspect, l'invention concerne une composition, avantageusement dermatologique et/ou cosmétique, préférentiellement protectrice cutanée vis-à-vis d'agents toxiques, caractérisée en ce qu'elle comprend de la Scléritine dans au moins un excipient ou véhicule adapté pour une application topique.

### BRÈVE DESCRIPTION DES FIGURES ET SEQUENCES

Les buts, objets, ainsi que les caractéristiques et avantages de l'invention ressortiront mieux de la description détaillée d'un mode de réalisation de cette dernière qui est illustré par les figures d'accompagnement suivantes dans lesquelles :
Figure 1 : schéma de la structure générale de la protéine Scléritine recombinante. Les vingt premiers acides aminés représentent le peptide signal, clivé lors du processus de maturation intracellulaire de la protéine (donc non présent dans la protéine mature sécrétée). Les six histidines en C-terminal de la protéine représentent le tag histidine (HISx6) utile pour le procédé de purification de la Scléritine recombinante.
Figure 2 : graphes illustrant la viabilité de lignées cellulaires a) Endothéliales, b) Kératinocytes, c) Fibroblastes, exposées à la Scléritine à différentes doses sur 3 jours.
Figure 3 : graphe illustrant la viabilité de cellules endothéliales humaines exposées à un agent toxique (l'imidazole à 150mM final) en présence de doses croissantes de Scléritine sur 6 jours.
Figure 4 : graphe issu de la figure 3 illustrant le nombre total de cellules vivantes au jour 6 suivant les conditions de la figure 3.
Figure 5 : Photos au microscope inversé à contraste de phase de fibroblastes humains soumis à des rayonnements ultraviolets au jour 1, a) et b) en présence de Scléritine à 4µg/ml, c) et d) en présence de BSA à 4µg/ml ou e) et f) en présence de PBS seul. (Grossissement de 80X pour les photos a, c et e du haut, et grossissement de 200X pour les photos b, d et f du bas)
Figure 6 : Photos au microscope inversé à contraste de phase de fibroblastes humains soumis à des rayonnements ultraviolets au jour 3 - a) et b) en présence de Scléritine à 4µg/ml, c) et d) en présence de BSA à 4µg/ml ou e) et f) en présence de PBS seul (grossissement de 200X pour les photos a, c et e du haut, et grossissement 400X pour les photos b, d et f du bas).
SEQ ID NO 1 : séquence nucléotidique naturelle de la Scléritine, comprenant 468 nucléotides, référencée dans le catalogue GenBank du NCBI sous le numéro JQ652458.
SEQ ID NO 2 : séquence de la Scléritine codon-optimisée, comprenant 486 nucléotides, obtenue par synthèse, pour une meilleure prise en charge transcriptionnelle par le système de production baculovirus/cellules d'insectes. De plus, elle possède un ajout de 18 paires de bases à son extrémité 3' codant pour un tag histidine. La séquence optimisée possède, hors tag histidine, une identité de 346 paires de bases sur 468 au total avec la séquence naturelle, soit une homologie nucléotidique de 74 %.
SEQ ID NO 3 : séquence protéique de 155 acides aminés de la Scléritine naturelle.
SEQ ID NO 4 : séquence protéique de la Scléritine recombinante codée à partir de la séquence optimisée SEQ ID NO 2. Hors tag histidine, elle possède 100 % d'homologie avec la séquence protéique naturelle.

### EXPOSE DETAILLE DE MODES DE REALISATION PARTICULIERS

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention concerne l'utilisation de la Scléritine en tant qu'agent protecteur de cellules contre des agents toxiques.

Avantageusement, suivant des variantes préférées, mais non limitatives, l'invention est telle que :
- les agents toxiques sont des agents chimiques ;
- les agents chimiques sont choisis parmi des substances inorganiques, des composés organiques volatils, des agents oxydants, des bases et acides forts, des composés imidazolés.
- les agents chimiques sont choisis parmi des agents chimiques d'origine biologique, parmi lesquels des toxines et venins animaux.
- les agents chimiques sont choisis parmi des agents cytotoxiques chimiothérapeutiques, parmi lesquels des agents alkylants, des antimétabolites, des agents alcaloïdes, des antibiotiques anticancéreux.
- les agents toxiques sont des agents physiques ;
- les agents physiques sont des rayonnements ultraviolets ;
- la Scléritine ne présente pas de cytotoxicité pour les cellules pour des doses inférieures ou égales à 4000ng/ml ;
- les cellules sont des cellules animales ;
- les cellules sont des cellules de la peau ;
- l'utilisation est faite pour la protection de la peau ;
- les cellules sont des fibroblastes, des cellules endothéliales ou des cellules épithéliales notamment des kératinocytes ;
- l'utilisation se fait par application topique ; la scléritine est sous une forme adaptée à une administration topique;
- la viabilité des cellules exposées à un agent toxique en présence de Scléritine est supérieure de 30 % à la viabilité des cellules exposées à un agent toxique seul ; c'est-à-dire en absence de Scléritine,
- la protéine de Scléritine présente une identité d'au moins 90 % avec la séquence protéique SEQ ID NO 3;
- la Scléritine est sous une forme adaptée à une application sur la peau avant l'exposition ou le contact avec un agent toxique.

Suivant un autre aspect, l'invention concerne une composition, avantageusement dermatologique et/ou cosmétique et préférentiellement protectrice cutanée vis-à-vis d'agents toxiques, caractérisée en ce qu'elle comprend de la Scléritine dans au moins un excipient ou véhicule adapté pour une application topique, par exemple sur la peau.

Selon une possibilité, la composition comprend une quantité de Scléritine inférieure ou égale à 4000ng/ml de milieu de culture de manière à ne pas présenter de cytotoxicité pour les cellules.

Suivant un autre aspect, l'invention concerne un procédé non thérapeutique de protection de la peau face à des agents toxiques caractérisé en ce qu'il comprend l'application sur la peau d'une composition comprenant de la Scléritine avant l'exposition ou le contact avec un agent toxique.

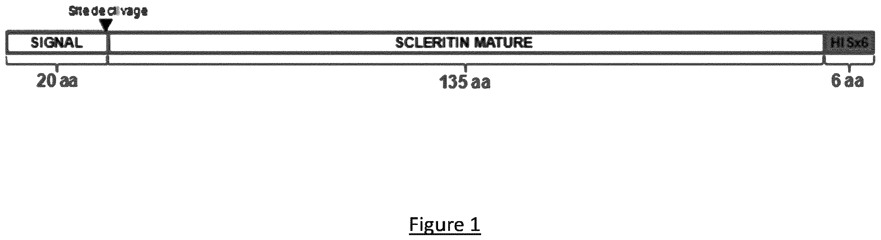

L'invention concerne la Scléritine qui est une protéine de 135 acides aminés. Cette protéine est codée par un gène de séquence de nucléotides SEQ ID No 1. La séquence d'acide aminé de la Scléritine est illustrée en regard de la séquence de nucléotide et reprise en figure 1. La Scléritine comprend un peptide signal de 20 acides aminés.

Selon l'invention, la Scléritine est produite par transfection de cellules de mammifères ou préférentiellement par système baculovirus / cellules d'insectes. La production de la Scléritine est par exemple réalisée suivant le procédé décrit dans la publication Buclez PO et al. "Rapid, scalable, and low-cost purification of recombinant adeno-associated virus produced by baculovirus expression vector system". Mol Ther Methods Clin Dev. 2016 May 11 ;3:16035. doi: 10.1038/mtm.2016.35. Pour la production, la Scléritine peut comprendre une étiquette de purification également appelée « tag » et par exemple un tag histidine comprenant 6 histidines, placé à la suite de la séquence d'acide aminé de la Scléritine, comme illustré à la figure 1. Par ailleurs, le gène de la Scléritine peut avoir subi une optimisation de ces codons d'origine pour une meilleure prise en charge transcriptionnelle dans l'environnement interne des cellules d'insectes (lignées cellulaires productrices de type Sf9), comme illustrée par la SEQ ID NO 2. Cette optimisation peut résulter d'une différence de séquences nucléotidiques pouvant atteindre 30 % comparativement à la séquence naturelle SEQ ID NO1.

L'invention s'étend à toutes les protéines comprenant au moins la séquence protéique SEQ ID NO 3 avec une identité de 90 % préférentiellement de 100 %.

Selon l'invention, la Scléritine joue un rôle protecteur des cellules face à des agents toxiques.

Avantageusement, on entend par agent protecteur des cellules soumises à un agent toxique, un produit permettant aux cellules de conserver une viabilité à 6 jours au moins égale à 60 %. L'agent protecteur permet de contrer au moins partiellement les effets néfastes de l'agent toxique. Ainsi, selon l'invention, la différence de viabilité des cellules au jour 6 est d'au moins 30 % entre les cellules soumises à un agent toxique en présence de Scléritine et les cellules soumises à un agent toxique sans Scléritine.

On entend par agents toxiques, des agents chimiques ou des agents physiques qui sont toxiques pour les cellules c'est-à-dire qu'ils les endommagent et notamment que la viabilité des cellules exposées à l'agent toxique est diminuée.

Parmi les agents chimiques, on entend cytotoxique, c'est-à-dire des substances qui sont toxiques pour les cellules, éventuellement jusqu'à les détruire.

A titre d'exemple, les agents toxiques sont choisis parmi au moins l'un des suivants :
- des agents chimiques, parmi lesquels des substances inorganiques, des composés organiques volatiles, des agents oxydants, des bases et acides forts, des composés imidazolés.
- des agents chimiques d'origine biologique, parmi lesquels des toxines et venins animaux
- des agents cytotoxiques chimiothérapeutiques, parmi lesquels des agents alkylants, des antimétabolites, des agents alcaloïdes, des antibiotiques anticancéreux
parmi les agents physiques, on entend les rayonnements notamment les rayonnements ultraviolets dont les UVA (c'est-à-dire les rayonnements électromagnétiques de longueurs d'ondes comprises entre 400 et 315 nm), les UVB (c'est-à-dire les rayonnements électromagnétiques de longueurs d'ondes comprises entre 315 et 280 nm) et les UVC (c'est-à-dire les rayonnements électromagnétiques de longueurs d'ondes comprises entre 280 et 100 nm).

Les cellules protégées par la Scléritine sont des cellules eucaryotes animales, notamment des cellules humaines.

Préférentiellement, la Scléritine est utilisée comme agent protecteur de la peau.

A titre d'exemple, les cellules animales comprennent les fibroblastes, les cellules épithéliales préférentiellement des kératinocytes ou les cellules endothéliales.

De manière surprenante, la Scléritine s'avère ne pas présenter de cytotoxicité envers lesdites cellules. C'est-à-dire que la Dose Létale médiane ou DL₅ₒ est supérieure à 4000ng/ml. Ces quantités sont des doses particulièrement élevées pour ce qui est de substances étrangères à la cellule. Cette absence de toxicité présente un avantage notable de sorte que la Scléritine peut être envisagée pour des utilisations cosmétiques et/ou dermatologiques.

Par ailleurs, la Scléritine présente un effet protecteur proportionnel à la dose administrée.

La Scléritine est avantageusement introduite dans une composition pouvant être à visée cosmétique et/ou dermatologique pour une protection préférentiellement cutanée vis-à-vis d'agents toxiques.

La composition est destinée à une application topique. On entend par topique que l'application est à visée locale. Préférentiellement, l'absorption se fait par la peau par voie cutanée ou transdermique, ou par les muqueuses par voie auriculaire, nasale, pulmonaire, vaginale / en intra-utérin ou oculaire.

La composition comprend au moins un excipient ou véhicule adapté pour une application topique. Selon le type de préparation désirée (pommades, crèmes, gels, pâtes, émulsions), la composition finale pourra comporter un ou plusieurs des excipients suivants : eau, vaseline blanche, paraffine (solide ou liquide), macrogols (ou polyéthylène glycol, PEG), vaseline hydratée au sesquioléate de sorbitane ou au monostéarate de glycérol (avec par exemple de l'acide sorbique comme conservateur), cétomacrogol tamponné (avec par exemple de l'acide sorbique comme conservateur), crème hydrophile anionique (avec ou sans glycérol), oléate de décyle, carbomères (avec par exemple de l'aminométhyl propanol comme stabilisateur), cérat réfrigérant (cire d'abeille blanche, cires d'esters cétyliques), huiles végétales (par exemple l'huile de sésame), oxyde de zinc (ZnO), talc, et tout autre excipient répondant favorablement à la pharmacopée européenne concernant les préparations pharmaceutiques.

### Exemple 1 : Effet Exogène de la Scléritine - cytotoxicité

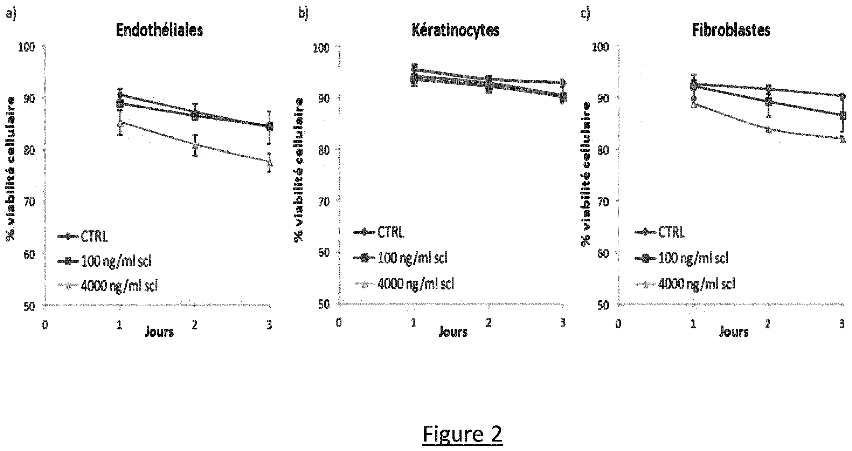

Différentes lignées cellulaires humaines : endothéliales, kératinocytes et fibroblastes, sont mises en contact avec des doses croissantes de Scléritine : 0, 100ng/ml et 4000ng/ml. Les résultats sont présentés sur les graphes de la figure 2.

Les cellules sont mises en culture dans les puits de plaque de culture. A t=24h les cellules sont mises en contact avec la Scléritine (formulée dans un tampon HEPES 50 mM, NaCl 50 mM, pH 7.8) qui est introduite dans le milieu de culture complet à des doses de 0, 100 ou 4000 ng de Scléritine par ml de milieu de culture.

Le jour de l"ajout de la Scléritine est considéré dans les exemples comme le jour 0.

Chaque jour les cellules d'un puits sont soumises au comptage cellulaire après protocole de décollement à la trypsine-EDTA, et comptage par compteur automatique (type Countess, Life Technology). La viabilité cellulaire est quantifiée sur lame par ajout de bleu de Trypan (colorant vital) à la suspension cellulaire.

On constate qu'à t=3 jours, la viabilité des cellules exposées à la Scléritine est similaire ou légèrement inférieure à celle des cellules contrôle non exposées.

Notamment, pour les cellules endothéliales au jour 3 : 78 % de viabilité pour les cellules exposées à 4000ng/ml de Scléritine contre 85 % de viabilité pour les cellules exposées à 100ng/ml de Scléritine ou non exposées.

Pour les kératinocytes au jour 3 : 90 % de viabilité pour les cellules exposées à 4000ng/ml et 100ng/ml de Scléritine contre 92 % de viabilité pour les cellules non exposées.

Pour les fibroblastes au jour 3 : 82 % de viabilité pour les cellules exposées à 4000ng/ml, 88 % de viabilité pour les cellules exposées à 100ng/ml et 90 % de viabilité pour les cellules non exposées.

En conclusion, aucune cytotoxicité cellulaire de la Scléritine sur des cellules primaires humaines n'est pas identifiable (jusqu'aux doses de Scléritine de 4000 ng/ml de milieu de culture).

### Exemple 2 : Effet Exogène de la Scléritine - protection contre des agents chimiques

Des cellules endothéliales humaines primaires sont mises en contact avec des doses croissantes de Scléritine 0,4µg/ml, 0,2 µg/ml, 0,1 µg/ml et 0,05 µg/ml et un agent toxique appartenant à la famille des dérivés imidazolés : l'imidazole (CAS N° 288-32-4).

Les cellules sont mises en culture dans les puits d'une plaque de culture. A t=24h les cellules sont mises en contact avec la Scléritine qui est introduite dans le milieu de culture complet aux différentes doses par ml de milieu de culture.

Le jour d'ajout de la Scléritine est considéré comme le jour 0. 24h après l'ajout de Scléritine, l'agent toxique (imidazole) est ajouté au milieu de culture pour une concentration finale dans le milieu de 150mM. L'ajout de l'agent toxique se fait au jour 1.

Chaque jour les cellules d'un puits sont soumises au comptage en suivant le protocole à la trypsine décrit précédemment.

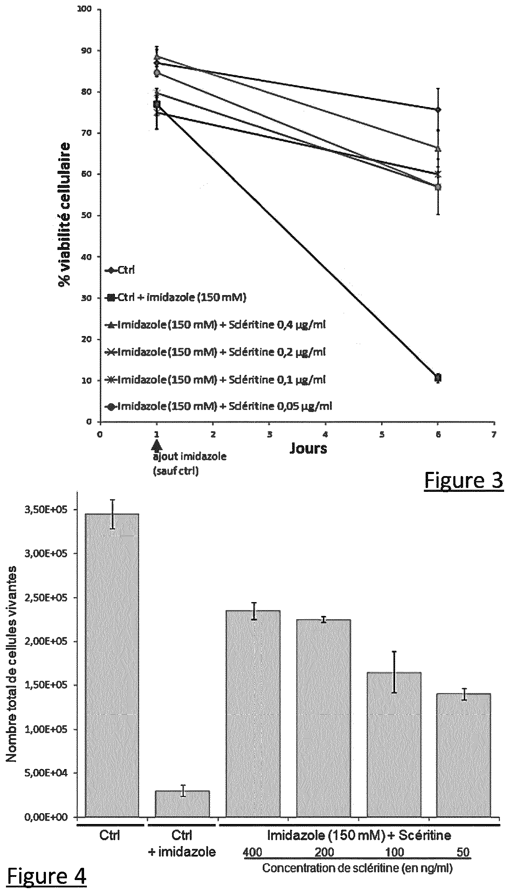

Les cellules sont observées sous microscope à contraste de phase et comptées, après protocole à la trypsine, chaque jour jusqu'au jour 6. Les résultats sont présentés sur les graphes de la figure 3.

On constate qu'au jour 6, la viabilité des cellules exposées à l'imidazole seul est proche de 10 % alors que celle des cellules exposées à l'imidazole en présence de Scléritine est comprise entre 58 et 68 % et que les cellules contrôle sans imidazole et sans Scléritine est de 78 %. Ces résultats sont illustrés en figure 3.

A la figure 4, le nombre de cellules vivantes au jour 6 est comparé selon les différentes conditions. La différence entre le nombre de cellules vivantes avec l'imidazole seul ou en présence de Scléritine est nettement visible.

La Scléritine joue donc un rôle protecteur des cellules face à l'imidazole.

### Exemple 3 : Effet Exogène de la Scléritine - protection contre des agents physiques

Des fibroblastes primaires humains sont exposés à des rayonnements ultraviolets en présence ou en absence de Scléritine.

Les cellules sont mises en culture dans les puits de plaque de culture. A t=24h, le milieu de culture est retiré et remplacé par du PBS 1X (pour éviter les potentiels effets d'atténuations des radiations UV par les protéines sériques solubles). Les cellules sont mises en contact avec la Scléritine dans du PBS 1X à une concentration finale de 4 µg/ml. Les cellules sont incubées 30 minutes à 37°C. Les cellules sont ensuite irradiées par des UltraViolets C à 254nm à une puissance de 1000µJ/cm². 5 minutes après l'irradiation, un milieu de culture complet est ajouté dans les puits et les cellules sont mises en culture dans un incubateur à 37°C et 5 % de CO₂.

Le jour d'ajout de la Scléritine est considéré comme le jour 0. L'irradiation aux UVC se fait également au jour 0.

Les cellules d'un puits sont contrôlées à J1 et J3.

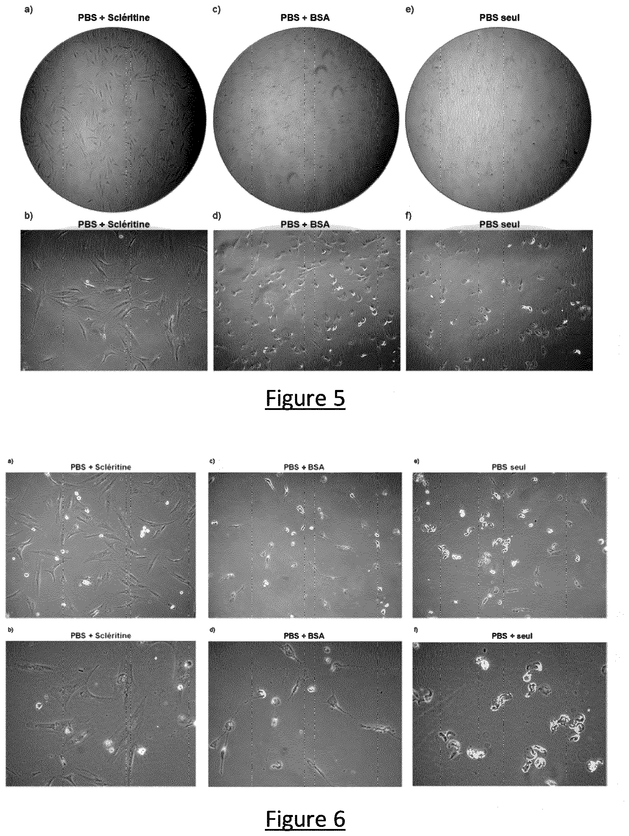

La figure 5 montre des photos au microscope inversé à contraste de phase (grossissement 80X pour les figures 5a, c et e, et grossissement 200X pour les figures 5b, d et f) de plaque de fibroblastes au jour 1 après irradiation en présence de 4µg/ml de Scléritine : figures 5 a) et b), de BSA à 4µg/ml : figures 5 c) et d) ou de PBS seul : figures 5 e) et f) .

On constate que les fibroblastes sont endommagés avec le PBS seul ou supplémenté en BSA alors que leur aspect reste correct pour les fibroblastes en présence de Scléritine.

La figure 6 montre des photos au microscope inversé à contraste de phase (grossissement 200X pour les figures 6a, c et e, et grossissement 400X pour les figures 6b, d et f) de plaques de fibroblastes au jour 3 après irradiation en présence de 4µg/ml de Scléritine : figures 6 a) et b), de BSA à 4µg/ml : figures 6 c) et d) ou de PBS seul : figures 6 e) et f).

On constate au jour 1 que les fibroblastes irradiés en présence de Scléritine présentent un aspect typique des fibroblastes, c'est-à-dire fusiforme ou étoilée avec des prolongements très fins. Avec la BSA seule, les fibroblastes sont condensés, les prolongements sont réduits voir absents. De même avec le PBS seul, les fibroblastes sont condensés et les prolongements sont réduits. Dès le jour 1, les fibroblastes irradiés en présence du PBS seul ou de BSA seule présentent un aspect typique des corps apoptotiques (mort cellulaire programmée). A ce stade, les tentatives de quantification des concentrations de cellules vivantes dans les condition avec BSA ou PBS seul échouent en raison d'absence de cellules comptabilisables, probablement fortement fragilisées par le protocole d'irradiation. Il peut être clairement conclu que les fibroblastes sont endommagés avec le PBS seul ou le BSA seul alors que leur aspect reste correct en présence de Scléritine.

Au jour 3, les constatations sont identiques et plus marquées encore ; les fibroblastes irradiés en présence de Scléritine présentent un aspect typique des fibroblastes, c'est-à-dire fusiforme ou étoilée avec des prolongements très fins. Avec le BSA seul, les fibroblastes sont condensés, les prolongements sont réduits voir absents. De même avec le PBS seul, les fibroblastes sont condensés et les prolongements sont absents. Les fibroblastes irradiés en présence du PBS seul ou du BSA seul présentent un aspect typique des corps apoptotiques. De même qu'au jour 1, les tentatives de quantification des concentrations de cellules vivantes dans les conditions avec BSA ou PBS seul échouent en raison d'absence de cellules comptabilisables, probablement fortement fragilisées par le protocole d'irradiation.

La Scléritine joue donc un rôle protecteur des cellules face à aux rayons ultraviolets C. Les ultraviolets C sont des rayonnements à faibles longueurs d'onde et sont donc extrêmement énergétiques. Cette énergie leur confère un pouvoir d'altération considérable sur les molécules biologiques. L'effet protecteur marqué de la Scléritine vis-à-vis des rayonnements UVC laisse penser que l'effet de la Scléritine vis-à-vis des rayonnements UVB et UVA, moins énergétiques, serait également très intéressant.

## Revendications

1. Scléritine pour son utilisation dans un traitement dermatologique en tant qu'agent protecteur pour des cellules contre des agents toxiques.

2. Scléritine pour son utilisation dans un traitement dermatologique selon la revendication précédente dans laquelle les agents toxiques sont des agents chimiques.

3. Scléritine pour son utilisation dans un traitement dermatologique selon la revendication précédente dans laquelle les agents chimiques sont choisis parmi des substances inorganiques, des composés organiques volatils, des agents oxydants, des bases et acides forts, des composés imidazolés.

4. Scléritine pour son utilisation dans un traitement dermatologique selon l'une quelconque des revendications 1 ou 2 dans laquelle les agents chimiques sont choisis parmi des agents chimiques d'origine biologique, parmi lesquels des toxines et venins animaux.

5. Scléritine pour son utilisation dans un traitement dermatologique selon l'une quelconque des revendications 1 ou 2 dans laquelle les agents chimiques sont choisis parmi des agents cytotoxiques chimiothérapeutiques, parmi lesquels des agents alkylants, des antimétabolites, des agents alcaloïdes, des antibiotiques anticancéreux.

6. Scléritine pour son utilisation dans un traitement dermatologique selon la revendication 1 dans laquelle les agents toxiques sont des agents physiques.

7. Scléritine pour son utilisation dans un traitement dermatologique selon la revendication précédente dans laquelle les agents physiques sont des rayonnements ultraviolets.

8. Scléritine pour son utilisation dans un traitement dermatologique selon l'une quelconque des revendications précédentes pour la protection de la peau.

9. Scléritine pour son utilisation dans un traitement dermatologique selon l'une quelconque des revendications précédentes pour laquelle les cellules sont des fibroblastes, des cellules endothéliales ou des cellules épithéliales, notamment des kératinocytes.

10. Scléritine pour son utilisation dans un traitement dermatologique selon l'une quelconque des revendications précédentes sous une forme adaptée à une administration topique.

11. Scléritine pour son utilisation dans un traitement dermatologique selon l'une quelconque des revendications précédentes dans laquelle la protéine de Scléritine présente une identité de séquence d'au moins 90 % avec la séquence protéique SEQ ID NO 3.

12. Scléritine pour son utilisation dans un traitement dermatologique selon l'une quelconque des revendications précédentes dans laquelle la Scléritine est sous une forme adaptée à une application sur la peau avant l'exposition ou le contact avec un agent toxique.

13. Composition **caractérisée en ce qu'**elle comprend de la Scléritine dans au moins un excipient ou véhicule adapté pour une application topique.

14. Composition selon la revendication précédente comprenant une quantité de Scléritine inférieure ou égale à 4000ng/ml de milieu de culture de manière à ne pas présenter de cytotoxicité pour les cellules.

## Patentansprüche

1. Skleritin zur Verwendung in einer dermatologischen Behandlung als Schutzagens für Zellen gegen toxische Agenzien.

2. Skleritin zur Verwendung in einer dermatologischen Behandlung nach dem vorstehenden Anspruch, wobei es sich bei den toxischen Agenzien um chemische Agenzien handelt.

3. Skleritin zur Verwendung in einer dermatologischen Behandlung nach dem vorstehenden Anspruch, wobei die chemischen Agenzien ausgewählt sind aus anorganischen Substanzen, flüchtigen organischen Verbindungen, oxidierenden Agenzien, starken Basen und Säuren, Imidazol-haltigen Verbindungen.

4. Skleritin zur Verwendung in einer dermatologischen Behandlung nach einem der Ansprüche 1 oder 2, wobei die chemischen Agenzien ausgewählt sind aus chemischen Agenzien biologischen Ursprungs, darunter Toxine und Tiergifte.

5. Skleritin zur Verwendung in einer dermatologischen Behandlung nach einem der Ansprüche 1 oder 2, wobei die chemischen Agenzien ausgewählt sind aus zytotoxischen chemotherapeutischen Agenzien, darunter alkylierende Agenzien, Antimetaboliten, alkaloiden Agenzien, Antibiotika gegen Krebs.

6. Skleritin zur Verwendung in einer dermatologischen Behandlung nach Anspruch 1, wobei es sich bei den toxischen Agenzien um physikalische Agenzien handelt.

7. Skleritin zur Verwendung in einer dermatologischen Behandlung nach dem vorstehenden Anspruch, wobei es sich bei den physikalischen Agenzien um ultraviolette Strahlen handelt.

8. Skleritin zur Verwendung in einer dermatologischen Behandlung nach einem der vorstehenden Ansprüche zum Schutz der Haut.

9. Skleritin zur Verwendung in einer dermatologischen Behandlung nach einem der vorstehenden Ansprüche, wobei es sich bei den Zellen um Fibroblasten, Endothelzellen oder Epithelzellen, insbesondere Keratinozyten, handelt.

10. Skleritin zur Verwendung in einer dermatologischen Behandlung nach einem der vorstehenden Ansprüche in einer Form, die für eine topische Verabreichung geeignet ist.

11. Skleritin zur Verwendung in einer dermatologischen Behandlung nach einem der vorstehenden Ansprüche, wobei das Skleritin-Protein eine Sequenzidentität von mindestens 90 % mit der Proteinsequenz SEQ ID-NR. 3 aufweist.

12. Skleritin zur Verwendung in einer dermatologischen Behandlung nach einem der vorstehenden Ansprüche, wobei das Skleritin in einer Form vorliegt, die für eine Anwendung auf der Haut vor der Exposition gegenüber oder dem Kontakt mit einem toxischen Agens geeignet ist.

13. Zusammensetzung, **dadurch gekennzeichnet, dass** sie Skleritin in mindestens einem Hilfsstoff oder Träger umfasst, der für eine topische Anwendung geeignet ist.

14. Zusammensetzung nach dem vorstehenden Anspruch, die eine Skleritin-Menge von kleiner oder gleich 4.000 ng/ml Kulturmedium umfasst, sodass sie keine Zytotoxizität für die Zellen aufweist.

## Claims

1. Scleritin for its use in a dermatological treatment as an agent for protecting cells against toxic agents.

2. Scleritin for its use in a dermatological treatment according to the preceding claim, wherein the toxic agents are chemical agents.

3. Scleritin for its use in a dermatological treatment according to the preceding claim, wherein the chemical agents are selected from inorganic substances, volatile organic compounds, oxidising agents, strong bases and acids, imidazole compounds.

4. Scleritin for its use in a dermatological treatment according to any one of claims 1 or 2, wherein the chemical agents are selected from chemical agents of biological origin, amongst which animal toxins and venoms.

5. Scleritin for its use in a dermatological treatment according to any one of claims 1 or 2, wherein the chemical agents are selected from chemotherapeutic cytotoxic agents, amongst which alkylating agents, antimetabolites, alkaloid agents, anticancer antibiotics.

6. Scleritin for its use in a dermatological treatment according to claim 1, wherein the toxic agents are physical agents.

7. Scleritin for its use in a dermatological treatment according to the preceding claim, wherein the physical agents are ultraviolet radiations.

8. Scleritin for its use in a dermatological treatment according to any one of the preceding claims, for skin protection.

9. Scleritin for its use in a dermatological treatment according to any one of the preceding claims, for which the cells are fibroblasts, endothelial cells or epithelial cells, in particular keratinocytes.

10. Scleritin for its use in a dermatological treatment according to any one of the preceding claims, in a form suited for a topical administration.

11. Scleritin for its use in a dermatological treatment according to any one of the preceding claims, wherein the Scleritin protein features at least 90% sequence identity with the protein sequence SEQ ID NO 3.

12. Scleritin for its use in a dermatological treatment according to any one of the preceding claims, wherein Scleritin is in a form suited for an application on the skin before exposure or contact with a toxic agent.

13. A composition **characterised in that** it comprises Scleritin in at least one excipient or vehicle suited for a topical application.

14. The composition according to the preceding claim, comprising an amount of Scleritin smaller than or equal to 4000 ng/ml of the culture medium so as to have no cytotoxicity for the cells.


### SEQUENCE LISTING

<110> SQY THERAPEUTICS CENTRE SCIENTIFIQUE DE MONACO
<120> UTILISATION DE LA SCLERITINE EN TANT QU'AGENT PROTECTEUR DE CELLULES CONTRE DES AGENTS TOXIQUES
<130> SCLERITINE
<160> 4
<170> BiSSAP 1.3.6
<210> 1
   <211> 468
   <212> DNA
   <213> Corallium rubrum
<400> 1
   atgaaatttt ttggtgcgat tttggtttgt ctacttcttg ccattccgta tggctcgcca      60
   cagcagcaaa ctgtagttat gaaacacggg tttattcggc acaggaaggt tggtcagggc     120
   gtaattaatc cattaagctc tgaagcagtg gcattgtttt tcaacaaaaa gtggaatact     180
   tttattgaac ttagcaaacg tatgcaacga gagtcatcca atttttgcag agcgaacttc     240
   ggagtcctta acgactggca aaaaagacag tgcagttgca tcagcatctt cagcttcatg     300
   aaccaaggaa gggatgcaat gctggtggcg agaacaactt ttggagaaat ttggcagaat     360
   tttaacaaat ttgggccaac tgaatactgc aacacacggc ctgtacagcc tatttccagg     420
   cagctagatg atctttgtta ctgtatgaca ggaaatccct ctgtttga                  468
<210> 2
   <211> 486
   <212> DNA
   <213> Artificial Sequence
<220>
<400> 2
   atgaagttct tcggtgctat cctcgtgtgc ctcctgctgg ctatccccta cggctccccg      60
   caacaacaga cggtcgtgat gaaacatggt ttcatccgcc accgtaaagt gggtcagggc     120
   gtcattaacc cactgtccag cgaggctgtc gccctcttct tcaacaagaa atggaacact     180
   ttcatcgagt tgtctaagag gatgcaaaga gaatcttcaa acttctgccg cgctaacttc     240
   ggcgttctga acgactggca gaaacgccaa tgcagttgta tctcgatttt ctccttcatg     300
   aaccagggac gcgatgcaat gctcgttgct cgtaccactt tcggagagat ctggcaaaac     360
   ttcaacaaat tcggtccgac agaatactgt aacacgaggc ctgtgcagcc catttccaga     420
   caactggacg acttgtgcta ctgtatgact ggaaacccat ccgttcacca ccaccaccat     480
   cactga                                                                486
<210> 3
   <211> 155
   <212> PRT
   <213> Corallium rubrum
<400> 3
   Met Lys Phe Phe Gly Ala Ile Leu Val Cys Leu Leu Leu Ala Ile Pro
   1               5                   10                  15
   Tyr Gly Ser Pro Gln Gln Gln Thr Val Val Met Lys His Gly Phe Ile
               20                  25                  30
   Arg His Arg Lys Val Gly Gln Gly Val Ile Asn Pro Leu Ser Ser Glu
           35                  40                  45
   Ala Val Ala Leu Phe Phe Asn Lys Lys Trp Asn Thr Phe Ile Glu Leu
       50                  55                  60
   Ser Lys Arg Met Gln Arg Glu Ser Ser Asn Phe Cys Arg Ala Asn Phe
   65                  70                  75                  80
   Gly Val Leu Asn Asp Trp Gln Lys Arg Gln Cys Ser Cys Ile Ser Ile
                   85                  90                  95
   Phe Ser Phe Met Asn Gln Gly Arg Asp Ala Met Leu Val Ala Arg Thr
               100                 105                 110
   Thr Phe Gly Glu Ile Trp Gln Asn Phe Asn Lys Phe Gly Pro Thr Glu
           115                 120                 125
   Tyr Cys Asn Thr Arg Pro Val Gln Pro Ile Ser Arg Gln Leu Asp Asp
       130                 135                 140
   Leu Cys Tyr Cys Met Thr Gly Asn Pro Ser Val
   145                 150                 155
<210> 4
   <211> 161
   <212> PRT
   <213> Artificial Sequence
<220>
   <223> sequence proteique de la scleritine recombinante codee a partir de la sequence optimisée de la SEQ ID NO 2
<400> 4
   Met Lys Phe Phe Gly Ala Ile Leu Val Cys Leu Leu Leu Ala Ile Pro
   1               5                   10                  15
   Tyr Gly Ser Pro Gln Gln Gln Thr Val Val Met Lys His Gly Phe Ile
               20                  25                  30
   Arg His Arg Lys Val Gly Gln Gly Val Ile Asn Pro Leu Ser Ser Glu
           35                  40                  45
   Ala Val Ala Leu Phe Phe Asn Lys Lys Trp Asn Thr Phe Ile Glu Leu
       50                  55                  60
   Ser Lys Arg Met Gln Arg Glu Ser Ser Asn Phe Cys Arg Ala Asn Phe
   65                  70                  75                  80
   Gly Val Leu Asn Asp Trp Gln Lys Arg Gln Cys Ser Cys Ile Ser Ile
                   85                  90                  95
   Phe Ser Phe Met Asn Gln Gly Arg Asp Ala Met Leu Val Ala Arg Thr
               100                 105                 110
   Thr Phe Gly Glu Ile Trp Gln Asn Phe Asn Lys Phe Gly Pro Thr Glu
           115                 120                 125
   Tyr Cys Asn Thr Arg Pro Val Gln Pro Ile Ser Arg Gln Leu Asp Asp
       130                 135                 140
   Leu Cys Tyr Cys Met Thr Gly Asn Pro Ser Val His His His His His
   145                 150                 155                 160
   His